# EUROPEAN PATENT APPLICATION

(11) **EP 2 644 590 A1**
(43) Date of publication of application: **02.10.2013**
(21) Application number: 12162507.3
(22) Date of filing: 30.03.2012
(51) Int. Cl.: C07C 209/56

(54) **Synthesis of 2-(3,4-difluorophenyl)cyclopropanamine derivatives and salts**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to the field of organic synthesis and describes the synthesis of specific intermediates suitable for the preparation of triazolopyrimidine compounds such as ticagrelor.

## Description

The present invention relates to the field of organic synthesis, in particular to the synthesis of specific intermediates suitable for the synthesis of triazolopyrimidine compounds.

An important triazolopyrimidine compound is ticagrelor (TCG; Brilinta®; 3-[7-[[(1*R*,2*S*)-2-(3,4-difluorophenyl)cyclopropyl]amino]-5-(propylthio)-*3H*-*1*,2,3-triazolo[4,5-d]pyrimidin-3-yl]-5-(2-hydroxyethoxy)-(1*S*,2*S*,3*R*,5S)-1,2-cyclopentanediol) having the following structural formula

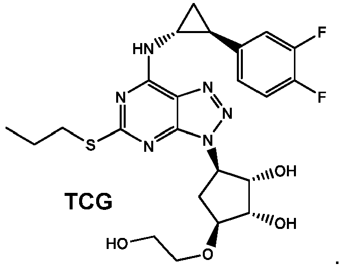

Ticagrelor shows pharmaceutical activity by functioning as a P2Y12 receptor antagonist and thus is indicated for the treatment or prevention of thrombotic events, for example stroke, heart attack, acute coronary syndrome or myocardial infection with ST elevation, other coronary artery diseases and arterial thrombosis as well as other disorders related to platelet aggregation (WO 00/34283).

All synthetic approaches mentioned above utilize as one of the key intermediates the intermediate **CPA** ((1*R*,2*S*)-2-(3,4-difluorophenyl)cyclopropylamine) of formula

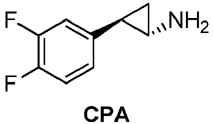

There are several synthetic paths for preparation of intermediate **CPA** known in the literature.

According to WO 01/92200 and WO 01/92263 **CPA** is prepared as shown in Scheme 1. 3,4-Difluorobenzaldehyde is reacted with malonic acid in the presence of pyridine and piperidine to yield (*E*)-3-(3,4-difluorophenyl)-2-propenoic acid, which is converted to (*E*)-3-(3,4-difluorophenyl)-2-propenoyl chloride by using thionyl chloride in the presence of toluene and pyridine. A solution of L-menthol in toluene is added to the obtained compound in the presence of pyridine to yield (1*R*,2*S*,5*R*)-2-isopropyl-5-methylcyclohexyl (*E*)-3-(3,4-difluorophenyl)-2-propenoate, which is with dimethylsulfoxonium methylide, sodium iodide and NaOH in DMSO converted to (1*R*,2*S*,5*R*)-2-isopropyl-5-methylcyclohexyl trans-2-(3,4-difluorophenyl)-cyclopropanecarboxylate. The latter is then hydrolyzed to (1*R*,2*R*)-2-(3,4-difluorophenyl)-cyclopropanecarboxylic acid, which is subsequently converted to (1*R*,2*R*)-2-(3,4-difluorophenyl)cyclopropanecarbonyl chloride with thionyl chloride. In the last two steps, the obtained carbonyl chloride is first converted to the corresponding azide by addition of sodium azide and *tert*-butylammonium bromide, which is finally converted to **CPA.**

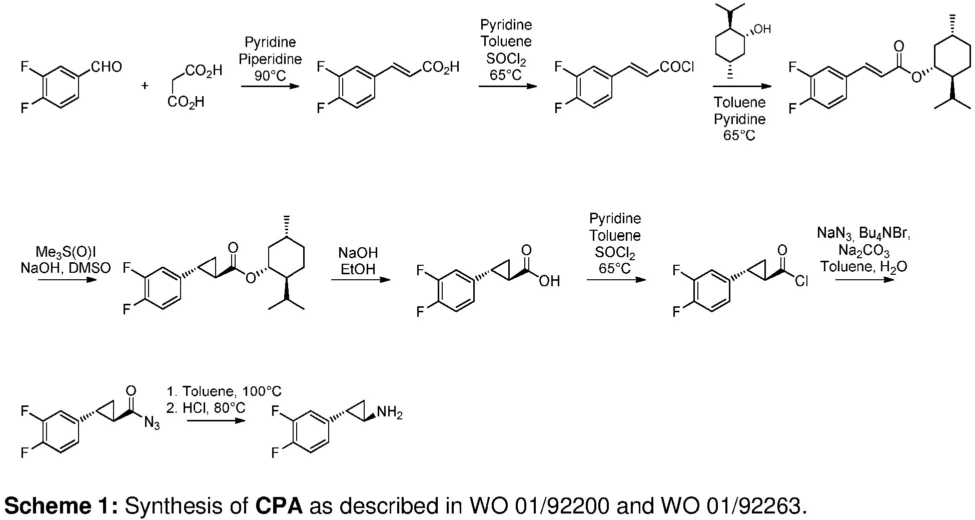

The eight steps synthesis described in Scheme 1 is very long and uses toxic compounds like sodium azide and pyridine. In addition, inorganic azides are potentially explosive. Moreover, the overall yield of the reaction is low.

Another process for the preparation of **CPA** is described in Bioorg. Med. Chem. Lett. 2007, 17, 6013-6018. The synthesis of the (1*R*,2*S*)-2-(3,4-difluorophenyl)cyclopropylamine begins with derivatisation of substituted cinnamic acid **A** with Oppolzer's sultam to give **B.** Diastereoselective cyclopropanation then provides, after recrystallisation, cyclopropylamide **C** in high chiral purity which is readily saponified to acid **D.** A four-step Curtius rearrangement gives **CPA.**

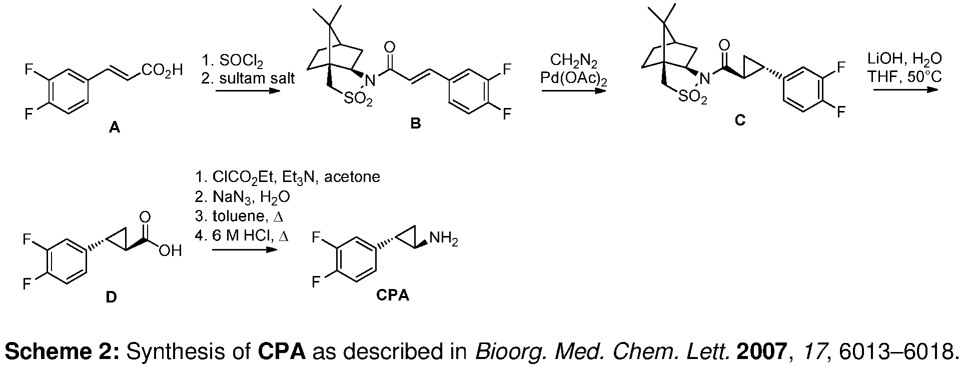

This 8 steps long synthesis presented in Scheme 2 involves the use of hazardous and explosive materials like sodium hydride, diazomethane and sodium azide. Moreover, highly expensive chiral sultam and palladium acetate are used. In addition, this lengthy process involves column chromatographic purifications, which are generally undesirable in view of large scale industrial applicability.

A process described in WO 11/017108 represents an improvement of the process described above in Scheme 2. Use of the most hazardous reagents has been omitted, *in situ* formation of diazomethane and expensive diphenylphosphoryl azide, (2*R*)-bornane-10,2-sultam and palladium acetate, however, still do not satisfy the need for safe and cheap industrial process. The overall approach of the improved process is presented in Scheme 3.

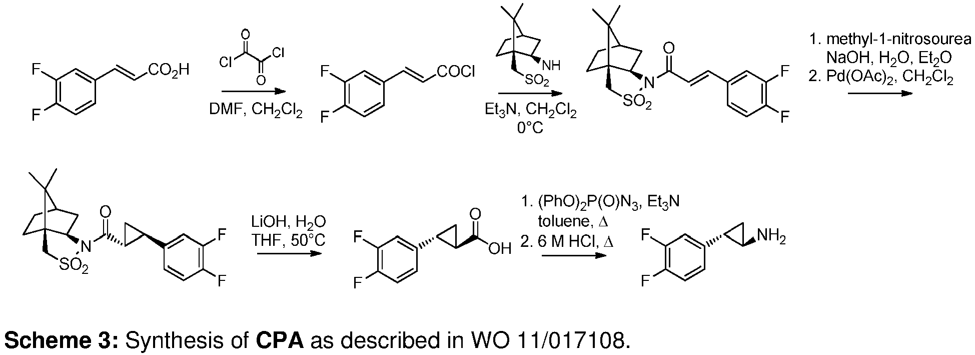

According to WO 08/018822 and WO 08/018823, **CPA** is prepared as shown in Scheme 4. First step involves reacting 1,2-difluorobenzene with chloroacetyl chloride in the presence of aluminium trichloride to produce 2-chloro-1-(3,4-difluorophenyl)ethanone. The keto group of the latter is then reduced by the use of chiral oxazaborolidine catalyst and borane dimethylsulfide complex to yield 2-chloro-1-(3,4-difluorophenyl)ethanol, which is then reacted with triethylphosphoacetate in the presence of sodium hydride in toluene to produce (1*R,*2*R)-*2-(3,4-difluorophenyl)cyclopropyl carboxylate. In the final two steps the ester compound is first converted to amide by methyl formate in the presence of ammonia, said amide is then reacted with sodium hydroxide and sodium hypochlorite to produce **CPA.**

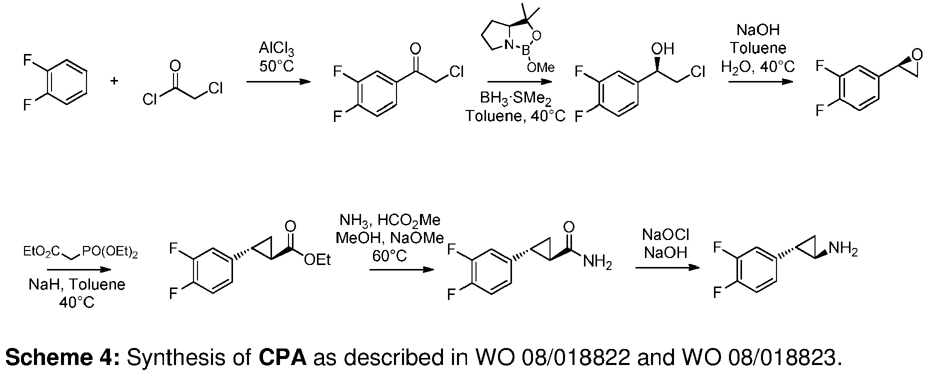

The disadvantages of the process described in WO 08/018822 and WO 08/018823 are the use of expensive chiral oxazaborolidine catalyst and toxic borane dimethylsulfide complex as well as use of explosive materials like sodium hydride.

Another synthetic route for preparing **CPA** is described in WO 11/132083 (Scheme 5). 1,2-Difluorobenzene is reacted with 3-chloropropionyl chloride to produce 3-chloro-1-(3',4'-difluorophenyl)-propan-1-one, which is by addition of *N*,*N-*dimethylformamide, phloroglucinol and sodium iodide in the next step converted to 1-(3',4'-difluorophenyl)-3-nitro-propan-1-one. The keto group of the latter intermediate is in the subsequent step stereochemically reduced to hydroxyl group by the use of chiral oxazaborolidine together with borane dimethyl sulfide or borane-*N*,*N-*diethyl aniline complex in the presence of tetrahydrofurane. The obtained (1*R*)-1-(3',4'-difluorophenyl)-3-nitro-propan-1-ol is then added to a mixture of triphenylphosphine and diethylazodicarboxylate in benzene to yield (1*S*,2*R*)-2-(3',4'-difluorophenyl)-1-nitrocyclopropane, which is in the last step converted to **CPA** by reduction of the nitro group by catalytic hydrogenation with palladium catalyst and zink dust.

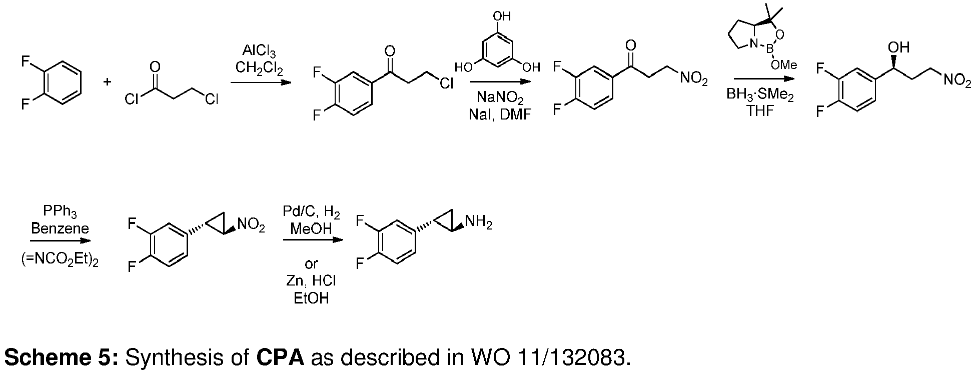

The disadvantage of the process described in WO 11/132083 is the use of expensive chiral oxazaborolidine and palladium catalyst, sodium iodide, toxic borane dimethylsulfide complex, heavy metals and hazardous diethyl azodicarboxylate.

WO 12/001531 describes another alternative synthetic path for preparation of **CPA** (Scheme 6). In this case the starting reagent 3,4-difluorobenzaldehide is reacted with a mixture of methyltriphenylphosphonium bromide, 1,8-diazabicyclo[5.4.0]undec-7-ene (DBU) and toluene to yield 3,4-difluorostyrene. The obtained intermediate is then added to dichloro(*p-*cymene)ruthenium(II) dimer and (*S*,*S*)-2,6-bis(4-isopropyl-2-oxazolin-2-yl)pyridine, which is followed by addition of ethyl diazoacetate to yield ethyl (1*R*,2*R*)-2-(3,4-difluorophenyl)-1-cyclopropanecarboxylate, which is converted to (1*R*,2*R*)-2-(3,4-difluorophenyl)-1-cyclopropanecarboxylic acid by hydrolysis in the presence of sodium hydroxide and methanol. The obtained carboxylic acid is with aqueous hydroxylamine solution further converted to (1*R*,2*R*)-2-(3,4-difluorophenyl)-1-cyclopropanecarboxamide, which is mixed with pyridine and acetic anhydride to yield (1*R*,2*R*)-*N*-(acetyloxy)-2-(3,4-difluorophenyl)-1-cyclopropane carboxamide. To the obtained intermediate in the presence of tetrahydrofurane the 1,8-diazabicyclo[5.4.0]undec-7-ene (DBU) is added, which is followed by addition of isopropyl acetate and ammonium chloride to finally yield **CPA.**

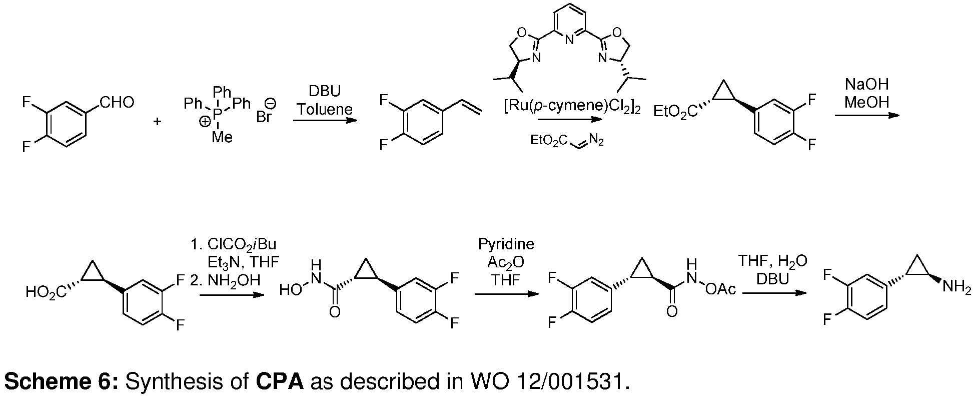

The disadvantage of the process described in WO 12/001531 is the use of expensive chiral ligand, dichloro(*p*-cymene)ruthenium(II) dimer, and use of toxic pyridine.

A major drawback of the hitherto known synthesis schemes for the preparation of **CPA** is that the synthesis is long and/or expensive, or that environmentally unfriendly reagents are used, which makes the prior art processes unsuitable for large scale preparation of **CPA.** In addition, in those cases, in which **CPA** is prepared via (1*R*,2*R*)-2-(3,4-difluorophenyl)-1-cyclopropanecarboxylic acid derivatives, a hazardous Curtius rearrangement conducted in the presence of sodium azide is used to convert carboxylic acid to amine. A need therefore remains for an alternative but commercially viable process for preparation of **CPA** which would at the same time be less hazardous and more environmentally friendly.

### Summary of the Invention

The object of the present invention was to provide an industrially applicable and economical process for obtaining (1*R*,2*S*)-2-(3,4-difluorophenyl)cyclopropylamine **(CPA),** an important intermediate in preparation of ticagrelor **(TCG).**

The present invention provides a process for the preparation of a compound of formula **IX** 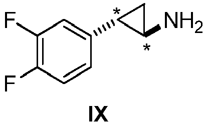 wherein the chiral center * is in its (*R*) or (*S*) configuration, comprising the steps of:
(i) providing a compound of formula **V** 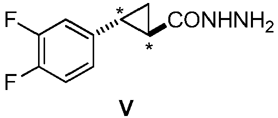 wherein the chiral center * is in its (*R*) or (*S*) configuration,
(ii) converting the hydrazide of formula **V** to an azide of formula **VI** 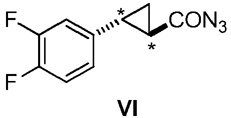 wherein the chiral center * is in its (*R*) or (*S*) configuration, and
(iii) converting the obtained compound of formula **VI** to provide the compound of formula **IX.**

The process defined above allows for preparation or synthesis of (1*R,*2*S)-*2*-*(3,4-difluorophenyl)cyclopropylamine with an industrially applicable and economical process while using environmentally friendly and non-explosive reagents. Preferred embodiments will be described below.

Furthermore, the present invention provides for isolation of crystalline (1*R,*2*S)-*2*-*(3,4-difluorophenyl)cyclopropylamine hydrochloride **(CPA**.HCl) without previous isolation of **CPA** base.

The present invention further provides a novel compound that is useful as an intermediate in the preparation of ticagrelor **(TCG).**

### Brief Description of the Figures

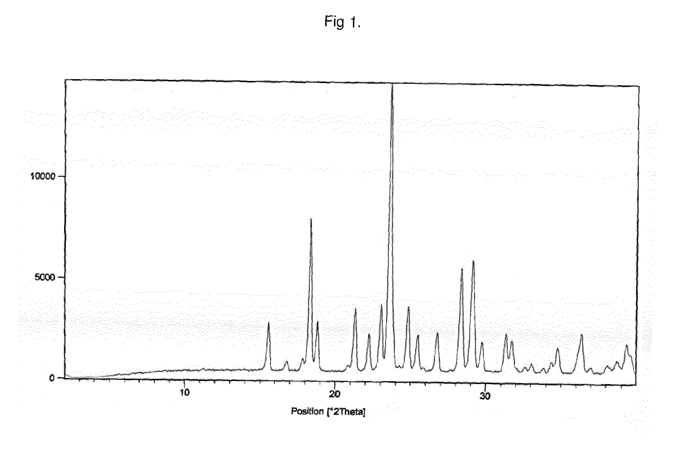

Fig. 1 shows an X-Ray diffraction pattern of crystalline (1*R*,2*S*)-2-(3,4-difluorophenyl)-cyclopropylamine hydrochloride **(CPA.**HCl) prepared according to example 18.

### Description of the Invention and of Preferred Embodiments

Aspects and preferred embodiments of the present invention will be described in further detail below, noting however that such aspects as well as embodiments and examples are presented for illustrative purposes only and shall not limit the invention in any way.

According to one embodiment, the azide compound of formula **VI** is converted to a compound of formula **IX** directly or through a compound of formula **VIII** as shown in Scheme 7.

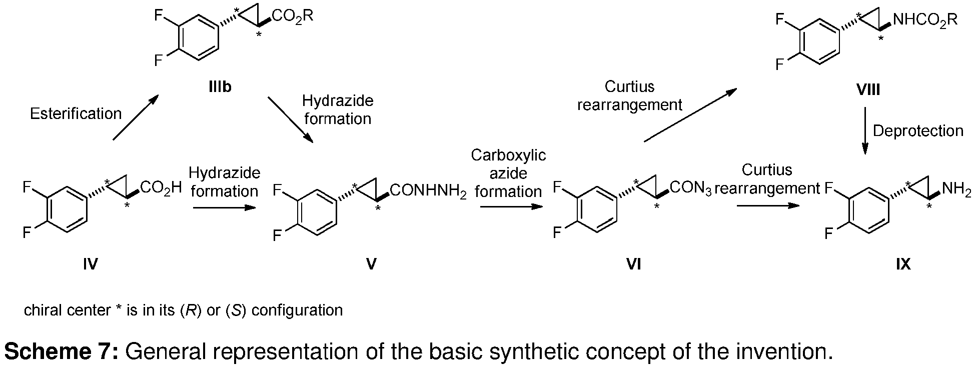

Alternatively, the compound of formula **VI** can be converted to a compound of formula **VIII** through isocyanate **VII** 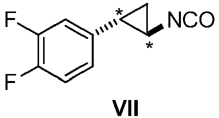 wherein the chiral center * is in its (*R*) or (*S*) configuration.

As presented in Scheme 7, the hydrazide **V** is prepared from trans-2-(3,4-difluorophenyl)-cyclopropanecarboxylic acid **IV** directly or optionally via ester compound **IIIb.**

According to another embodiment of the present invention, trans-2-(3,4-difluorophenyl)-cyclopropanecarboxylic acid **IV** is prepared as shown in Scheme 8.

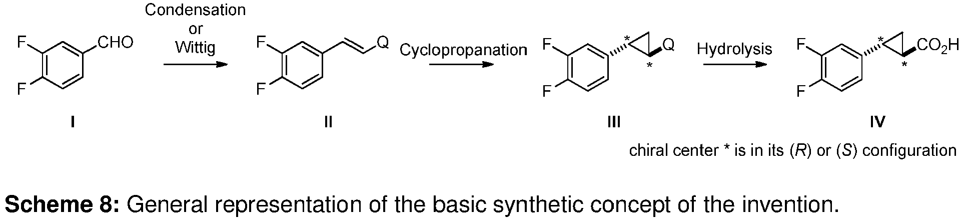

The process is based on cyclopropanation of alkene **II,** which can be prepared from 3,4-difluorobenzaldehyde **I through** aldol condensation or Wittig reaction. The cyclopropanation is carried out with a suitable reagent, for example with trimethylsulfoxonium iodide. The substituent Q in compounds of formula **II** and **III** can be any group that can be converted to either carboxylic group and allows cyclopropanation of **II.** Preferably, Q is selected from the group selected of C₁-C₆-alkyl carboxylic ester, carboxylic ester with a chiral alcohol such as L-menthol, *N*-substituted or unsubstituted carboxamide, cyano, hydroxymethyl, formyl, aldehyde, trihalomethyl, imide such as phthalimide etc. Cyclopropane **III** is then hydrolysed under acidic or basic conditions or oxidized to give *trans*-2-(3,4-difluorophenyl)cyclopropanecarboxylic acid **IV.**

The process of Scheme 8 selectively leads to trans-substituted racemic cyclopropanes. The enantiomers can be separated from the obtained racemic mixture in any step of preparation of compound **IX,** using principles in the art such as chromatographic separation, or crystallisation technics after preparation of diastereoisomers by introduction of a chiral moiety or by preparation diastereoisomeric salts with chiral counterion. For example, if Q is COO(L-menthyl) the racemic compound **III** can be separated to enantiomers by crystallisation as described in WO 01/092200. In this case (1*R*,2*S*,5*R*)-2-isopropyl-5-methylcyclohexyl-2-(3,4-difluorophenyl)-cyclopropanecarboxylate is then hydrolysed to (1*R*,2*R*)-2-(3,4-difluorophenyl)cyclopropanecarboxylic acid ((1*R*,2*R*) enantiomer of compound **IV).**

In a particularly preferred embodiment presented in Scheme 9, the synthesis of *trans-*2-(3,4-difluorophenyl)-cyclopropanecarboxylic acid **IV** begins with reacting 1,2-difluorobenzaldehyde and acetonitrile in the presence of a base, preferably KOH or BuLi, to give (*E*)-3-(3,4-difluorophenyl)acrylonitrile **IIa** as a white solid. **IIa** is then transformed to **IIIb** by cyclopropanation using trimethylsulfoxonium iodide and NaH or NaOH in DMSO. Enantiomerically enriched **IIIa** can be formed from **IIa** using methods for asymmetric cyclopropanation as described in the literature. In the next step, **IIIa** is hydrolyzed using basic or acidic conditions, preferably base, most preferably LiOH, to give trans-2-(3,4-difluorophenyl)-cyclopropanecarboxylic acid **IV.**

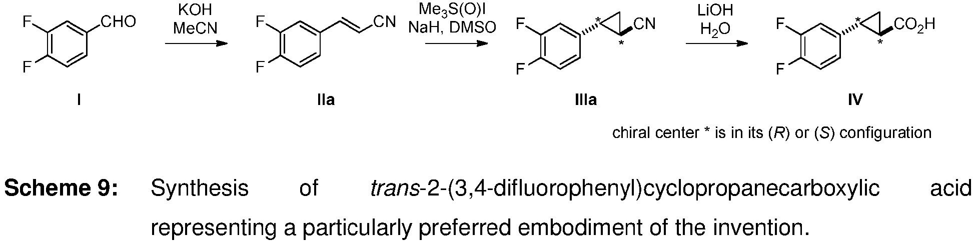

In another preferred embodiment, methyl *trans*-2-(3,4-difluorophenyl)cyclopropanecarboxylate **IIIb** is prepared from 1,2-difluorobenzaldehyde (Scheme 10). In the first step (*E*)-3-(3,4-difluorophenyl)acrylic acid **IIb** is prepared according to the procedure described in WO 01/92200 and WO 01/92263. This is followed by esterification, for which any acid in MeOH can be used, preferably H₂SO₄, to give **IIc** as white solid, which is subsequently cyclopropanated using trimethylsulfoxonium iodide and NaH or NaOH in DMSO. Again, the enantiomerically enriched **IIIb** can be formed from **IIc** using methods for asymmetric cyclopropanation as described in the literature.

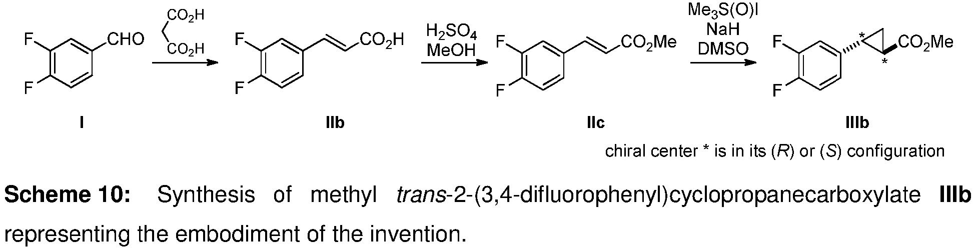

In the next step, the carboxylic acid **IV** is transformed to trans-2-(3,4-difluorophenyl)-cyclopropanecarbonyl azide **VI** (Scheme 11). **IV** can be converted directly to hydrazide **V** or through ester **IIIb,** which can be prepared by cyclopropanation of **IIc** as described above, or by esterification of **IV** using any acid in MeOH, preferably H₂SO₄.

Hydrazide **V** can be prepared as a white solid compound directly from the carboxylic acid **IV** by heating it with SOCI₂ in any aprotic solvent, preferably toluene, followed by reaction with hydrazine. Alternatively, hydrazide **V** is prepared by stirring ester **IIIb** and hydrazine in any solvent, preferably solvent from C₁-C₆ alcohols, most preferably methanol, in a temperature range from 0 to 150°C, preferably the temperature is 70°C.

Hydrazide **V** can be then reacted with nitrite, preferably NaNO₂ in acidic media, preferably in AcOH, to form *trans*-2-(3,4-difluorophenyl)cyclopropanecarbonyl azide **VI.**

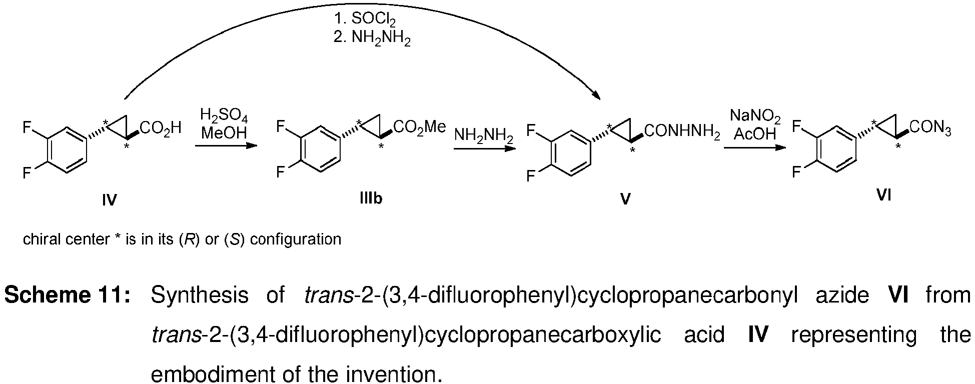

The procedure of Scheme 11 does not use explosive inorganic or organic azides. Furthermore, the intermediate hydrazide of formula **V** is solid and can be optionally purified by crystallisation if needed.

As discussed above, the preferred enantiomer can be obtained from the racemic mixture in any step of preparation of compound **IX** using principles well known in the art. In a preferred embodiment, the carboxylic acid of formula **IV** is provided in enantiomerically pure form (1*R*,2*R*)-2-(3,4-difluorophenyl)cyclopropanecarboxylic acid **(IV').** Use of enantiomerically pure carboxylic acid **IV'** gives (1*R*,2*R*)-2-(3,4-difluorophenyl)cyclopropanecarbonyl azide (compound **VI')** according to Scheme 12.

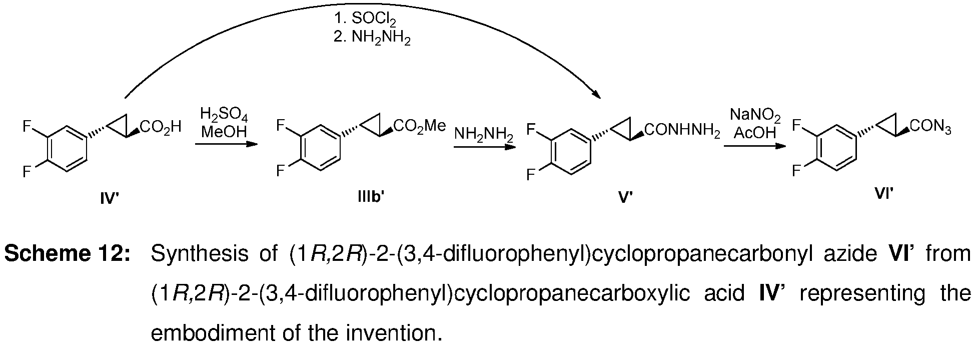

The enantiomerically pure (1*R*,2*R*)-2-(3,4-difluorophenyl)cyclopropanecarbonyl azide **VI'** or racemic *trans*-2-(3,4-difluorophenyl)cyclopropanecarbonyl azide **VI** can then be directly transformed to (1*R*,2*S*)-2-(3,4-difluorophenyl)cyclopropanamine **(CPA)** or *trans-2-(3,4-*difluorophenyl)cyclopropanamine **IX,** respectively, by a one-pot reaction as described in WO 01/92200 and WO 01/92263.

Alternatively, the carbamate protected amine **VIII** can also be prepared directly from **VI** or through isocyanate **VII** (Scheme 13), which can be prepared by simple heating of compound **VI** in any aprotic solvent, preferably toluene, at a temperature falling into the range of 25 to 150°C, preferably the temperature is 80°C. **VII** is then transformed to **VIIIa** by reaction with *t*BuOH at 25 to 150°C, preferably at the temperature of reflux, or by reacting KO*t*Bu in any aprotic solvent at a temperature falling in the range of -50 to 100°C, preferably at 0°C.

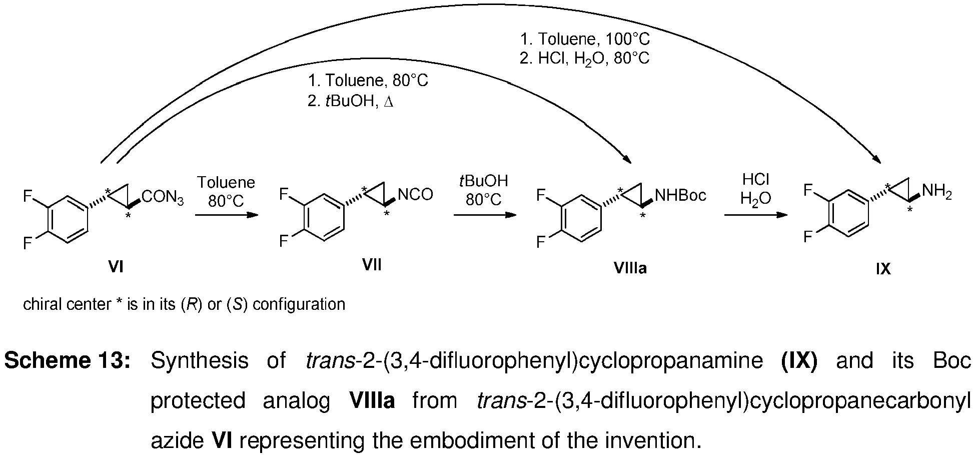

Analogously, in a preferred embodiment presented in Scheme 14 the enantiomerically pure chiral azide (1*R*,2*R*)-2-(3,4-difluorophenyl)cyclopropanecarbonyl **(VI')** is converted to (1*R*,2*R*)-2-(3,4-difluorophenyl)cyclopropanamine hydrochloride **(CPA)**

**VIIIa** or **VIIIa'** can also be prepared through one-pot transformation starting from **VI** or **VI',** respectively, according to the same reaction conditions as described above.

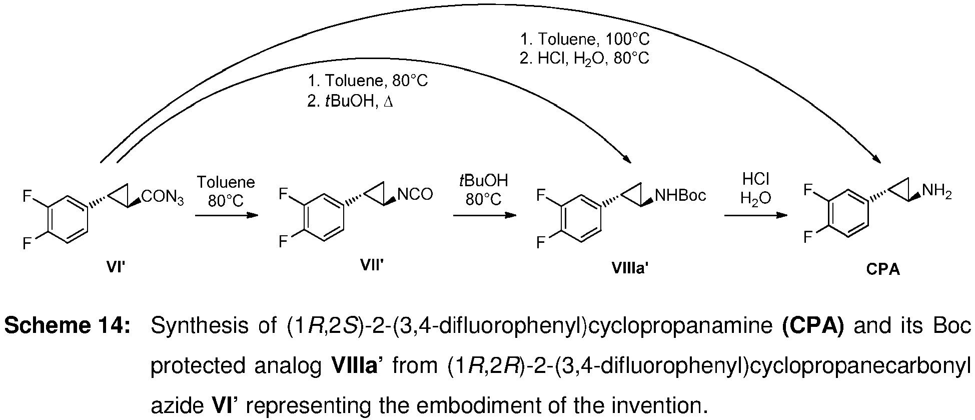

The intermediate **VIIIa** or **VIIIa'** is hydrolysed by hydrochloric acid in the mixture of methanol and water. This mixture is preferred due to unexpected lower solubility of hydrochloride salt in water. Said hydrochloride salt is provided in crystalline form and smoothly precipitates from the mixture, giving the product in high yield. Such approach is advantageous over the prior art process described in CN102249929, in which hydrolysis occurs in ethyl acetate/water mixture and the product is finally isolated following the neutralization, as a base from the organic phase.

The crystalline hydrochloride salt of **CPA** can be prepared also in the absence of water by introducing gaseous hydrogen chloride, optionally dissolved in an organic solvent, preferably diethyl ether, to a solution comprising **CPA** base in an organic solvent such as aromatic hydrocarbons or ethers.

Further aspect of the present invention resides in the provision of a valuable intermediate *trans-*2-(3,4-difluorophenyl)cyclopropanecarbohydrazide (compound of formula **V),** useful in the synthesis of ticagrelor **(TCG):** 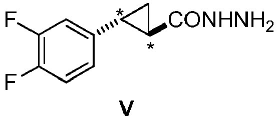 wherein the chiral center * is in its (*R*) or (*S*) configuration.

In a preferred embodiment the intermediate is enantiomerically pure (1*R*,2*R*)-2-(3,4-difluorophenyl)cyclopropanecarbohydrazide **(V')**

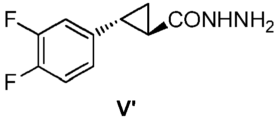

Yet further aspect of the present invention resides in the provision of crystalline *trans*-2-(3,4-difluorophenyl)cyclopropanamine hydrochloride **(IX.**HCl), wherein the chiral center * is in its (*R*) or (*S*) configuration.

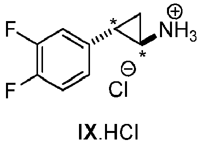

Further particularly preferred aspect of the present invention resides in the provision of crystalline (1*R*,2*S*)-2-(3,4-difluorophenyl)cyclopropanamine hydrochloride **(CPA.**HCl).

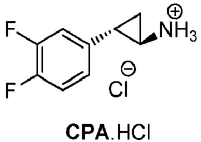

In the following the present invention will be described in further detail by illustrative, nonlimiting examples.

### Examples

### Example 1: Preparation of (E)-3-(3,4-difluorophenyl)acrylonitrile (IIa)

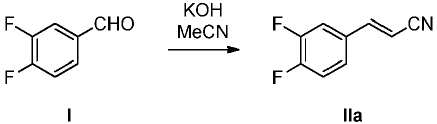

To a mixture of KOH (3.3 g, 50 mmol, 85%) and MeCN (40 mL) was heated to 80°C, and then **I** (7.1 g, 50 mmol) was added. The resulting reaction mixture was stirred at 80°C for 1 h, then it was poured into ice (50 g), and water (100 mL) was then added. After extraction with MeTHF (3 x 50 mL), combined organic layers were dried over MgSO₄, and then concentrated to afford crude product, which was then recrystallized from hexane to give title compound **IIa** as colorless crystals (1.67 g, 20% yield). DSC (Onset): 70°C; ¹H NMR (CDCl₃): 5.83 (d, 1H, *J* 16.7), 7.19-7.26 (m, 2H), 7.26-7.35 (m, 2H); ¹⁹F NMR (CDCl₃): -136.6 (m, 1F), -133.2 (m, 1F); MS (ESI) *m*/*z*: 166 [MH]⁺.

### Example 2: Preparation of (E)-methyl 3-(3,4-difluorophenyl)acrylate (IIc)

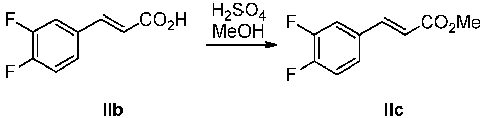

The compound **IIb** was prepared through procedure described in (WO 2001/92200 and WO 2001/92263).

A mixture of **IIb** (20.0 g, 109 mmol) and H₂SO₄ (96%, 0.50 g) in MeOH (150 mL) was stirred at 60°C for 3 days, then half of the MeOH was evaporated, 20 mL saturated aq. solution of NaHCO₃ was added, and water (50 mL) was added. White precipitate was then filtered off, washed with water and dried to afford title compound **IIc** as white powder (20.7 g, 90% yield). DSC (Onset): 76°C;¹H NMR (CDCl₃): 3.82 (s, 3H), 6.36 (d, 1H, *J* 16.0), 7.19 (m, 1H), 7.26 (m, 1H), 7.35 (m, 1H), 7.60 (d, 1H, *J* 16.0); MS (ESI) *m*/*z*: 199 [MH]⁺.

### Example 3: Preparation of trans-2-(3,4-difluorophenyl)cyclopropanecarbonitrile (IIIa)

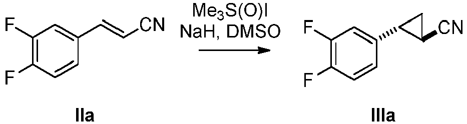

To a solution of trimethylsulfoxonium iodide (0.80 g, 3.64 mmol) in dry DMSO (10 mL) was added at 25°C NaH (60%, 0.15 g, 3.64 mmol). The resulting reaction mixture was stirred 30 min at 25°C, then **IIa** (0.50 g, 3.03 mmol) was added at 0°C, and reaction mixture was stirred at 25°C for 16 h. Water (80 mL) was added, and product was extracted to MeTHF (3 x 20 mL). Combined organic layers were dried over MgSO₄, and then concentrated to afford crude product, which was then purified by chromatography (SiO₂, hexane:EtOAc) to give title compound **IIIa** as colorless oil (0.29 g, 53% yield). ¹H NMR (CDCl₃): 1.41 (m, 1H), 1.54 (m, 1H), 1.65 (m, 1H), 2.61 (m, 1H), 6.87 (m, 1H), 6.92 (m, 1H), 7.11 (m, 1H); MS (ESI) *m*/*z*: 213 [MH]⁺.

### Example 4: Preparation of methyl trans-2-(3,4-difluorophenyl)cyclopropanecarboxylate (IIIb)

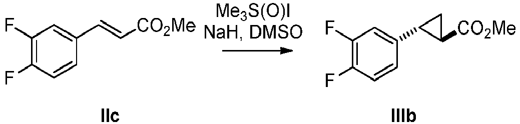

To a solution of trimethylsulfoxonium iodide (5.55 g, 25.2 mmol) in dry DMSO (40 mL) was added at 25°C NaH (60%, 1.0 g, 25.2 mmol). The resulting reaction mixture was stirred 30 min at 25°C, then **IIc** (5.0 g, 25.2 mmol) was added at 0°C, and reaction mixture was stirred at 25°C for 16 h. Water (200 mL) was added, and product was extracted to MeTHF (3 x 50 mL). Combined organic layers were dried over MgSO₄, and then concentrated to afford crude product, which was then purified by chromatography (SiO₂, hexane:EtOAc) to give title compound **IIIb** as colorless oil (4.0 g, 75% yield). ¹H NMR (CDCl₃): 1.26 (m, 1H), 1.60 (m, 1H), 1.85 (m, 1H), 2.49 (m, 1H), 3.72 (s, 3H), 6.83 (m, 1H), 6.89 (m, 1H), 7.06 (m, 1H); ¹⁹F NMR (CDCl₃): -137.8 (m, 1F), -140.8 (m, 1F); MS (ESI) *m*/*z*: 213 [MH]⁺.

### Example 5: Preparation of trans-2-(3,4-difluorophenyl)cyclopropanecarboxylic acid (IV)

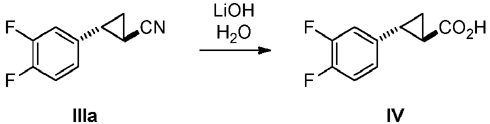

A mixture of **IIIa** (1.1 g, 6.14 mmol) and 4 M aq. LiOH (35 mL) in *i*PrOH (20 mL) was stirred at reflux temperature for 3.5 hours. Slowly 10% aq. HCI (70 mL) was added, and product was extracted to MeTHF (3 x 20 mL). Combined organic layers were dried over MgSO₄, and then concentrated to afford title compound **(IV)** as white solid (1.15 g, 95% yield). ¹H NMR (CDCl₃): 1.38 (m, 1H), 1.69 (m, 1H), 1.88 (m, 1H), 2.59 (m, 1H), 6.87 (m, 1H), 6.93 (m, 1H), 7.11 (m, 1H), 11.94 (br s, 1H); ¹⁹F NMR (CDCl₃): -137.5 (m, 1F), -140.4 (m, 1F); MS (ESI) *mlz:* 199 [MH]⁺.

### Example 6: Preparation of methyl trans-2-(3,4-difluorophenyl)cyclopropanecarboxylate (IIIb)

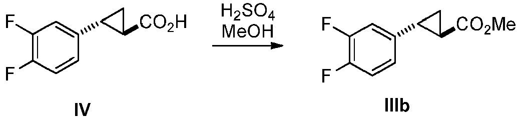

To a solution of **IV** (2.0 g, 10.1 mmol) in MeOH (40 mL) H₂SO₄ (0.2 g, 96%) was added at 0°C. The resulting reaction mixture was stirred at 60°C for 2 h, then saturated solution of NaHCO₃ (5 mL) was added, and product was extracted to THF (3 x 20 mL). Combined organic layers were dried over MgSO₄, and then concentrated to afford title compound **(IIIb)** as colorless oil (2.1 g, 98% yield). ¹H NMR (CDCl₃): 1.26 (m, 1H), 1.60 (m, 1H), 1.85 (m, 1H), 2.49 (m, 1H), 3.72 (s, 3H), 6.83 (m, 1H), 6.89 (m, 1H), 7.06 (m, 1H); ¹⁹F NMR (CDCl₃): -137.8 (m, 1F), -140.8 (m, 1F); MS (ESI) *mlz*: 213 [MH]⁺.

### Example 7: Preparation of trans-2-(3,4-difluorophenyl)cyclopropanecarbohydrazide (V)

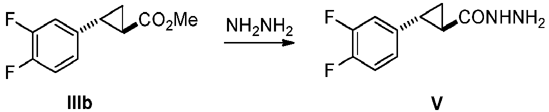

To a solution of **IIIb** (2.1 g, 9.90 mmol) in MeOH (15 mL) NH₂NH₂ (1.74 mL, 52% in water) was added at 25°C. The resulting reaction mixture was stirred at 70°C for 16 h, then 1 M NaOH (50 mL) was added, product (V) was filtered off, washed with water (50 mL), and dried in vacuum. White powder was obtained (1.8 g, 86% yield). DSC (Onset): 128.5°C; ¹H NMR (DMSO-d₆): 1.24 (m, 1H), 1.34 (m, 1H), 1.76 (m, 1H), 2.27 (m, 1H), 4.22 (br s, 2H), 6.98 (m, 1H), 7.20 (m, 1H), 7.30 (m, 1H), 9.18 (br s, 1H); ¹⁹F NMR (DMSO-d₆): -140.0 (m, 1F), -143.6 (m, 1F); MS (ESI) *mlz*: 213 [MH]⁺.

### Example 8: Preparation trans-2-(3,4-difluorophenyl)cyclopropanecarbonyl azide (VI)

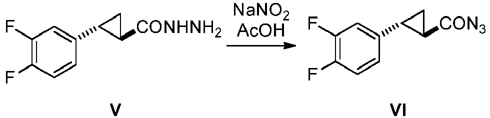

To a solution of **V** (1.0 g, 4.71 mmol) in AcOH (10 mL) was added under stirring at 25°C NaNO₂ (0.36 g, 5.18 mmol). Resulting reaction mixture was stirred at 25°C for 30 min, then water (100 mL) was added, product was extracted to EtOAc (3 x 30 mL), combined organic layers were dried over MgSO₄, then concentrated to afford crude compound, which was then purified by chromatography (SiO₂, hexane:EtOAc) to afford title compound **(VI)** as colorless oil (0.65 g, 62% yield). ¹H NMR (CDCl₃): 1.40 (m, 1H), 1.72 (m, 1H), 1.86 (m, 1H), 2.60 (m, 1H), 6.84 (m, 1H), 6.89 (m, 1H), 7.08 (m, 1H); ¹⁹F NMR (CDCl₃): -138.3 (m, 1F), -141.1 (m, 1F).

### Example 9: Preparation of 1,2-difluoro-4-(trans-2-isocyanatocyclopropyl)benzene (VII)

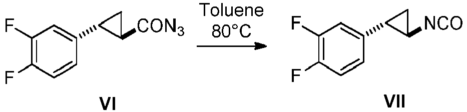

A solution of **VI** (1.31 g, 5.88 mmol) in toluene (10 mL) was stirred at 80°C for 2 h. Evaporation of the solvent afforded title compound **VII** as colorless oil (1.07 g, 93% yield).¹H NMR (CDCl₃): 1.20 (m, 1H), 1.33 (m, 1H), 2.18 (m, 1H), 2.90 (m, 1H), 6.81 (m, 1H), 6.86 (m, 1H), 7.09 (m, 1H); ¹⁹F NMR (CDCl₃): -137.6 (m, 1F), -140.8 (m, 1F).

### Example 10: Preparation of tert-butyl trans-2-(3,4-difluorophenyl)cyclopropyl)carbamate (VIIIa)

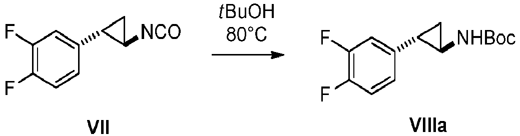

A solution of **VII** (1.0 g, 5.12 mmol) in *t*BuOH (15 mL) was stirred at 80°C for 24 h. Then water (50 mL) was added, and white solid material was filtered off and dried to afford title compound **VIIIa** as white powder (1.20 g, 87% yield). ¹H NMR (CDCl₃): 1.08-1.17 (m, 2H), 1.45 (s, 9H), 2.01 (m, 1H), 2.64 (m, 1H), 4.89 (br s, 1H), 6.89 (m, 1H), 6.96 (m, 1H), 7.03 (m, 1H); ¹⁹F NMR (CDCl₃): -139.3 (m, 1F), -142.8 (m, 1F); MS (ESI) *m*/*z*: 270 [MH]⁺.

### Example 11: Preparation of trans-2-(3,4-difluorophenyl)cyclopropanamine hydrochloride salt (IX.HCl)

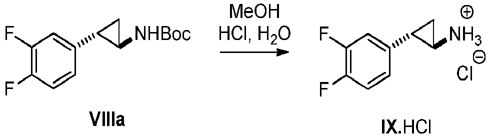

To a solution of **VIIIa** (0.15 g, 0.56 mmol) in MeOH (3 mL) was added 2 mL of 37% HCI. Resulting reaction mixture was stirred at 25°C for 2 h, then water (5 mL) was added, white precipitate was filtered off, washed with water (2 mL), and dried to afford title compound **IX** as HCI salt. DSC (Onset): 153°C; ¹H NMR (DMSO-d₆): 1.20 (m, 1H), 1.45 (m, 1H), 2.40 (m, 1H), 2.78 (m, 1H), 7.05 (m, 1H), 7.25 (m, 1H), 7.32 (m, 1H), 8.76 (br s, 3H); ¹⁹F NMR (DMSO-d₆): - 138.9 (m, 1F), -142.1 (m, 1F).

### Example 12: Preparation of methyl (1R,2R)-2-(3,4-difluorophenyl)cyclopropanecarboxylate (IIIb')

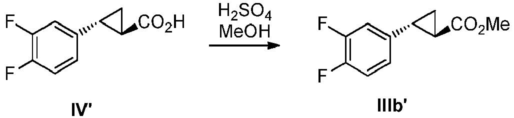

The compound **IV'** was prepared through procedure described in WO 2001/92200 and WO 2001/92263.

To a solution of **IV'** (4.0 g, 20.2 mmol) in MeOH (60 mL) H₂SO₄ (0.2 g, 96%) was added at 0°C. The resulting reaction mixture was stirred at 60°C for 2 h, then saturated solution of NaHCO₃ (10 mL) was added, and product was extracted to THF (3 x 30 mL). Combined organic layers were dried over MgSO₄, and then concentrated to afford title compound **(IIIb')** as colorless oil (3.94 g, 92% yield). ¹H NMR (CDCl₃): 1.26 (m, 1H), 1.60 (m, 1H), 1.85 (m, 1H), 2.49 (m, 1H), 3.72 (s, 3H), 6.83 (m, 1H), 6.89 (m, 1H), 7.06 (m, 1H); ¹⁹F NMR (CDCl₃): -137.8 (m, 1F), -140.8 (m, 1F); MS (ESI) *m*/*z*: 213 [MH]⁺.

### Example 13: Preparation of (1R,2R)-2-(3,4-difluorophenyl)cyclopropanecarbohydrazide (V')

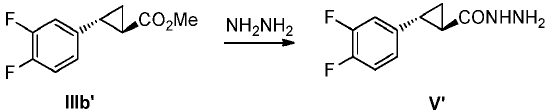

To a solution of **IIIb'** (3.15 g, 14.9 mmol) in MeOH (25 mL) NH₂NH₂ (2.61 mL, 52% in water) was added at 25°C. The resulting reaction mixture was stirred at 70°C for 16 h, then 1 M NaOH (70 mL) was added, product **(V')** was filtered off, washed with water (100 mL), and dried in vacuum. White powder was obtained (2.7 g, 86% yield). Melting point: 129°C; ¹H NMR (500 MHz, DMSO-d₆): 1.24 (m, 1H), 1.34 (m, 1H), 1.76 (m, 1H), 2.27 (m, 1H), 4.22 (br s, 2H), 6.98 (m, 1H), 7.20 (m, 1H), 7.30 (m, 1H), 9.18 (br s, 1H); ¹⁹F NMR (470 MHz, DMSO-d₆): -140.0 (m, 1F), -143.6 (m, 1F); MS (ESI) *m*/*z*: 213 [MH]⁺.

### Example 14: Preparation (1R,2R)-2-(3,4-difluorophenyl)cyclopropanecarbonyl azide (VI')

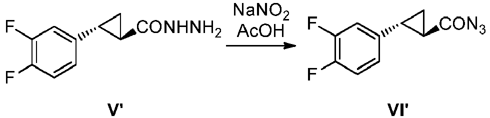

To a solution of **V'** (1.7 g, 8.0 mmol) in AcOH (15 mL) was added under stirring at 25°C NaNO₂ (0.61 g, 8.8 mmol). Resulting reaction mixture was stirred at 25°C for 30 min, then water (200 mL) was added, product was extracted to MeTHF (3 x 30 mL), combined organic layers were dried over MgSO₄, then concentrated to afford crude compound, which was then purified by chromatography (SiO₂, hexane:EtOAc) to afford title compound **(VI')** as colorless oil (1.32 g, 74% yield).¹H NMR (CDCl₃): 1.40 (m, 1H), 1.72 (m, 1H), 1.86 (m, 1H), 2.60 (m, 1H), 6.84 (m, 1H), 6.89 (m, 1H), 7.08 (m, 1H); ¹⁹F NMR (CDCl₃): -138.3 (m, 1F), -141.1 (m, 1F).

### Example 15: Preparation of tert-butyl (1R,2S)-2-(3,4-difluorophenyl)cyclopropyl)carbamate (VIIIa')

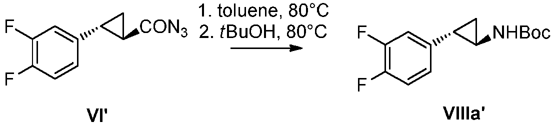

A solution of **VI'** (2.6 g, 11.6 mmol) in toluene (20 mL) was stirred at 80°C for 16 h. Then volatile components were evaporated and *t*BuOH (10 mL) was added. Resulting reaction mixture was stirred at 80°C for 6 days. Then water (50 mL) was added, and white solid material was filtered off and dried to afford title compound **VIIIa**' as white powder (1.72 g, 55% yield). DSC (Onset): 110°C;¹H NMR (CDCl₃): 1.08-1.17 (m, 2H), 1.45 (s, 9H), 2.01 (m, 1H), 2.64 (m, 1H), 4.89 (br s, 1H), 6.89 (m, 1H), 6.96 (m, 1H), 7.03 (m, 1H); ¹⁹F NMR (CDCl₃): -139.3 (m, 1F), -142.8 (m, 1F); MS (ESI) *m*/*z*: 270 [MH]⁺.

### Example 16: Preparation of (1R,2S)-2-(3,4-difluorophenyl)cyclopropanamine hydrochloride salt (CPA.HCl)

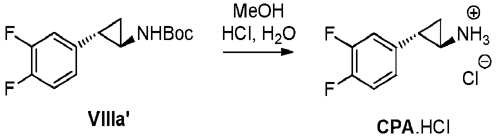

To a solution of **VIIIa'** (0.30 g, 1.12 mmol) in MeOH (10 mL) was added 4 mL of 37% HCI. Resulting reaction mixture was stirred at 25°C for 2 h, then MeOH was partly evaporated, water (10 mL) was added, white precipitate was filtered off, washed with water (5 mL), and dried to afford title compound **CPA** as HCI salt (0.18 g, 76% yield). DSC (Onset): 200°C; ¹H NMR (DMSO-d₆): 1.20 (m, 1H), 1.45 (m, 1H), 2.40 (m, 1H), 2.78 (m, 1H), 7.05 (m, 1H), 7.25 (m, 1H), 7.32 (m, 1H), 8.76 (br s, 3H); ¹⁹F NMR (DMSO-d₆): -138.9 (m, 1F), -142.1 (m, 1F).

### Example 17: Preparation of (1R,2S)-2-(3,4-difluorophenyl)cyclopropanamine (CPA)

To a 1 M NaOH (20 mL) was added under stirring at 25°C **CPA.**HCl (0.15 g, 0.78 mmol). Resulting reaction mixture was stirring at 25°C for 15 min, then the product was extracted to MeTHF (3 x 5 mL). Combined organic layers were dried over Na₂SO₄, then concentrated to afford CPA as yellowish oil (125 mg, 95% yield). ¹H NMR (CDCl₃): 0.88 (m, 1H), 1.02 (m, 1H), 1.66 (br s, 2H), 1.79 (m, 1H), 2.46 (m, 1H), 6.68-6.77 (m, 2H), 6.98 (m, 1H); ¹⁹F NMR (CDCl₃): -139.6 (m, 1F), -143.8 (m, 1F);

### Example 18: Preparation of (1R,2S)-2-(3,4-difluorophenyl)cyclopropanamine hydrochloride salt (CPA.HCl)

**CPA** was prepared as above or through procedure described in WO 2001/92200 and WO 2001/92263. To an ice-cold 37% HCI (30 mL) was slowly added (1*R*,2*S*)-2-(3,4-difluorophenyl)-cyclopropanamine **(CPA,** 5.0 g, 29.6 mmol). Resulting reaction mixture was stirred at 0°C, for 15 min, then white precipitate was filtered off, washed with water (10 mL) and dried. Filtrate was concentrated to ~50% of starting volume, white precipitate was again filtered off and dried. White crystals were obtained (5.28 g, 87% yield). XPRD, characteristic angles (2θ, in °): 15.6 ± 0.2, 18.4 ± 0.2, 21.3 ± 0.2, 23.1 ± 0.2, 23.6 ± 0.2, 28.4 ± 0.2, 29.1 ± 0.2, 31.3 ± 0.2; DSC (Onset): 200°C; ¹H NMR (DMSO-d₆): 1.20 (m, 1H), 1.45 (m, 1H), 2.40 (m, 1H), 2.78 (m, 1H), 7.05 (m, 1H), 7.25 (m, 1H), 7.32 (m, 1H), 8.76 (br s, 3H); ¹⁹F NMR (DMSO-d₆): -138.9 (m, 1F), -142.1 (m, 1F).

### Example 19: Preparation of (1R,2S)-2-(3,4-difluorophenyl)cyclopropanamine hydrochloride salt (CPA.HCl)

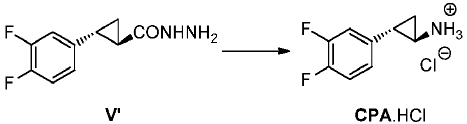

To a stirred mixture of (1*R*,2*R*)-2-(3,4-difluorophenyl)cyclopropanecarbohydrazide (V') (1.67 g, 7.87 mmol), toluene (30 mL), MeTHF (10 mL) and 2 M HCI (aq., 30 mL) at 20°C was added NaNO₂ (0.60 g, 8.66 mmol). Resulting reaction mixture was stirred at 20°C for 1 h, then water layer was separated and organic layer was dried over MgSO₄. Salts were filtered off and filtrate was stirred while heating at 100 °C for 1 h. Reaction mixture was then cooled to 20°C and it was slowly added to a stirred mixture of 37% HCI (aq., 30 mL) and water (10 mL) at 60°C. When all of the solution was added, the reaction mixture was stirred at 60°C for additional 15 min, then it was cooled to 20°C, and organic layer was separated. Water layer was then washed with MeTHF/toluene mixture (2:1, 3 x 30 mL) and neutralized with Na₂CO₃. **CPA** was then extracted to the toluene (2 x 20 mL), combined organic layers were dried over Na₂SO₄, salts were filtered off, and to the filtrate HCI (1 M solution in diethyl ether, 7 mL) was added. White precipitate was filtered off, washed with toluene (2 x 5 mL) and dried to afford title compound **(CPA.**HCl) as white powder (0.71 g, 44% yield). ¹H NMR (DMSO-d₆): 1.20 (m, 1H), 1.45 (m, 1H), 2.40 (m, 1H), 2.78 (m, 1H), 7.05 (m, 1H), 7.25 (m, 1H), 7.32 (m, 1H), 8.76 (br s, 3H); ¹⁹F NMR (DMSO-d₆): -138.9 (m, 1F), -142.1 (m, 1F).

Analytical data in examples were obtained with the following hardware:
X-Ray powder diffraction method:
   Conditions for obtaining powder X-ray diffraction (XRD) patterns: The powder X-ray diffraction patterns were obtained by methods known in the art using Philips X'Pert PRO diffractometer with X'Celerator detector using CuKα radiation (tube operating at 45 kV and 40 mA) in the Bragg-Brentano (reflection) geometry. Data were recorded from 2 to 40 ^{o}2θ in steps of 0.033 ^{o}2θ and the measurement time of 50 seconds per step. Variable divergence and antiscatter slits were used to maintain 12 mm of sample length irradiated.

Differential Scanning Calorimetry:
Conditions for obtaining DSC thermograms: Thermograms were obtained with Mettler Toledo DSC822e differential scanning calorimeter. The sample (1-10 mg) was placed in an unsealed aluminium pan with a hole and heated at 10°C/min in the temperature range from 30 °C to 250 °C.

NMR analysis:
All the NMR spectra were taken with Bruker Avance III 500 MHz spectrophotometer. ¹H NMR spectra were obtained at 500 MHz and ¹⁹F NMR spectra were obtained at 470 MHz at 25°C in CDCl₃ (7.24 ppm in ¹H spectrum) or DMSO-d₆ (2.50 ppm in ¹H spectrum). Chemical shifts are reported in ppm downfield from an internal TMS standard or relative to the residual solvent signal. Coupling constants (*J*) are given in hertz (Hz).

HPLC-MS analysis:
MS spectra were recorded with LC-MS system composed of Waters Alliance HPLC and Micromass Quattro micro mass spectrometer equipped with electrospray ionisation source.

## Claims

1. A process for the preparation of a compound of formula **IX** or a salt thereof 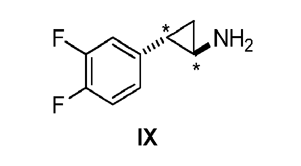 wherein the chiral center * is in its (*R*) or (*S*) configuration, comprising the steps of:
(i) providing a compound of formula **V** 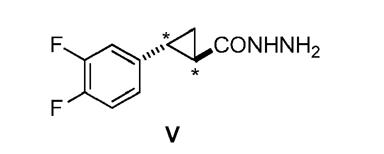 wherein the chiral center * is in its (*R*) or (*S*) configuration,
(ii) converting the hydrazide of formula **V** to an azide of formula **VI** 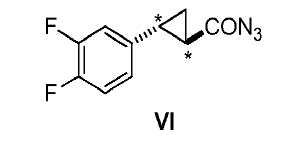 wherein the chiral center * is in its (*R*) or (*S*) configuration, and
(iii) converting the obtained compound of formula **VI** to provide the compound of formula **IX** or a salt thereof.

2. The process according to claim 1, wherein the compound of formula **VI** obtained in step (ii) is first converted to a compound of formula **VIII** 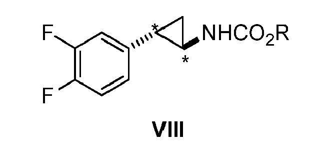 wherein the chiral center * is in its (*R*) or (*S*) configuration, and wherein R is linear or branched C₁-C₆ alkyl,
said compound **VIII** being converted to the compound of formula **IX.**

3. The process according to claim 2, wherein the compound of formula **VI** is first converted to a
compound of formula **VII** 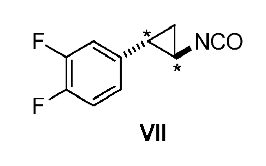 wherein the chiral center * is in its (*R*) or (*S*) configuration,
said compound **VII** being converted to the compound of formula **VIII.**

4. The process according to claim 2 or claim 3, wherein the process is conducted as a one-pot process.

5. The process according to claim 1, wherein the compound of formula **V** is prepared by a process comprising:
(i) providing a compound of formula **IIIb** or **IV** 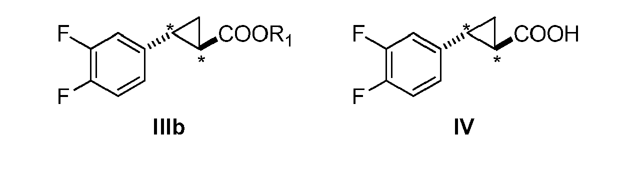 wherein the chiral center * is in its (*R*) or (*S*) configuration and wherein R₁ is linear or branched C₁-C₆ alkyl, and
(ii) converting the compound of formula **IIIb** or **IV** to the compound of formula **V.**

6. The process according to claim 5, wherein the compound of formula **IV** is first converted to a compound of formula **IIIb,** which is then converted to the compound of formula **V.**

7. The process according to claim 5, wherein the compound of formula **IV** is prepared by a process comprising:
(i) providing a compound of formula **I** 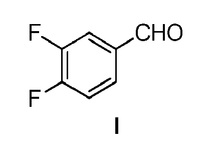
(ii) converting the compound of formula **I** to give an alkene of formula **II** 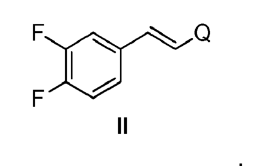 wherein Q is a group convertible to carboxyl group, ester group, amine or amine salt,
(iii) converting the obtained alkene of formula **II** by reaction of cyclopropanation to give a
compound of formula **III** 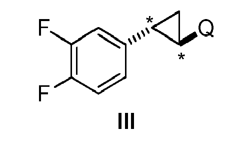 wherein the chiral center * is in its (*R*) or (*S*) configuration and wherein Q is as defined above,
(iv) converting the obtained compound of formula **III** to the compound of formula **IV** 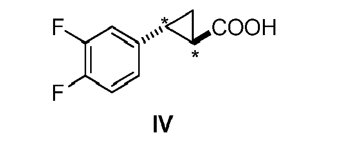 wherein the chiral center * is in its (*R*) or (*S*) configuration, and
(v) optionally separating the obtained *trans*-racemate of **IV** to provide the enantiomerically pure (1*R*,2*R*)-2-(3,4-difluorophenyl)cyclopropanecarboxylic acid.

8. The process according to claim 5, wherein the compound of formula **IV** is the enantiomerically pure (1*R*,2*R*)-2-(3,4-difluorophenyl)cyclopropanecarboxylic acid, the process comprising:
(i) providing a compound of formula I 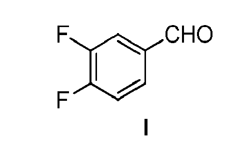
(ii) converting the compound of formula **I** to give an alkene of formula **II** 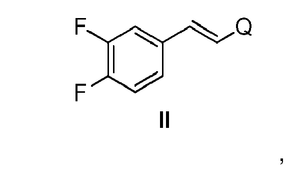 wherein Q is a group convertible to carboxyl group, ester group, amine or amine salt,
(iii) converting the obtained alkene of formula **II** by reaction of cyclopropanation to give a compound of formula **III** 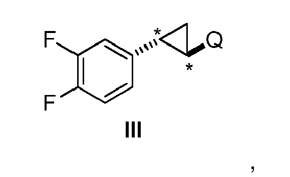 wherein the chiral center * is in its (*R*) or (*S*) configuration and wherein Q is as defined above,
(iv) separating the obtained trans-racemate of **III** to provide the enantiomerically pure compound of formula **III',** and 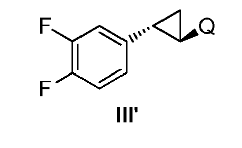
(v) converting the obtained compound of formula **III'** to (1*R,2R*)-2-(3,4-difluorophenyl)cyclopropanecarboxylic acid.

9. A compound of the following formula **V** and stereochemical isomeric forms thereof.

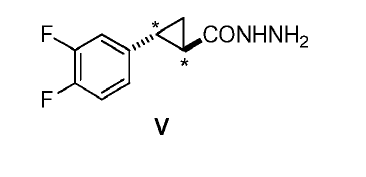

10. The process according to claim 1, wherein the salt of IX is a crystalline hydrochloride salt.

11. A crystalline hydrochloride salt of the following formula

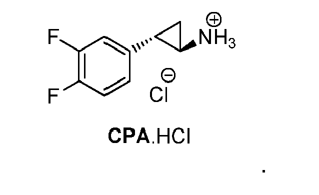

12. Use of any of the compounds of claim 9 to 11 in the preparation of ticagrelor.
